# EUROPÄISCHE PATENTSCHRIFT

(11) **EP 3 641 621 B1**
(45) Veröffentlichungstag und Bekanntmachung des Hinweises auf die Patenterteilung: **14.07.2021**
(21) Anmeldenummer: 18765356.3
(22) Anmeldetag: 20.08.2018
(51) Int. Cl.: A61B 1/00

(54) **ENDOSKOP-VORRICHTUNG**
ENDOSCOPE DEVICE
DISPOSITIF ENDOSCOPIQUE

(30) Priorität: 29.08.2017 DE 102017008148; 15.09.2017 DE 202017004822 U
(43) Veröffentlichungstag der Anmeldung: 29.04.2020
(73) Patentinhaber: Joimax GmbH, 76227 Karlsruhe (DE)
(72) Erfinder: RIES, Wolfgang, 76351 Linkenheim (DE); GERMANN, Martin, 76327 Pfinztal (DE)
(74) Vertreter: Lichti - Patentanwälte Partnerschaft mbB
(86) Internationale Anmeldenummer: PCT/EP2018/000405
(87) Internationale Veröffentlichungsnummer: WO 2019/042578

(56) Entgegenhaltungen:
- EP-A1- 2 213 220
- EP-A1- 2 269 532
- EP-A2- 0 894 473
- US-A- 5 997 473
- US-A1- 2011 201 921
- US-A1- 2014 039 258

## Beschreibung

Die Erfindung betrifft eine Endoskop-Vorrichtung mit einem proximalen Einführkopf und mit einem sich von diesem distal erstreckenden Schaft mit einer Mittelachse, mit mindestens seinem sich durch die Vorrichtung erstreckenden länglichen Lumen nach dem Oberbegriff des Anspruchs 1.

Minimal-invasive Operationen werden heute bereits mittels navigationsgestützter Operationsverfahren durchgeführt. Es werden hierzu unterschiedliche Navigationssysteme eingesetzt. Es kommen aktive und passive Systeme zum Einsatz. Bei aktiven Systemen ist ein in den Körper eines Patienten eingebrachtes Teil, wie ein Instrument oder chirurgisches Werkzeug mit einem Sender versehen, über den sich extern die Position des Instruments oder Werkzeugs, insbesondere des distalen am Eingriffsort befindlichen Endes bestimmen lässt. Bei passiven Systemen wird ein Feld erzeugt, welches über einen Sensor erfasst wird, wodurch wiederum die Position und Ausrichtung des Instruments oder chirurgischen Werkzeugs, insbesondere dessen distales Ende, direkt oder indirekt erfasst werden können. Direkte Erfassung des distalen Endes eines chirurgischen Teils beinhaltet die Anordnung des Sensors am distalen Ende des Teils selbst; indirekte Erfassung beinhaltet die feste starre Anbringung des Sensors in einer definierten Stelle, insbesondere Axialposition, an dem chirurgischen Teil. Aufgrund des gemessenen Sensorsignals können Rückschluss auf die Position und gegebenenfalls Ausrichtung des distalen Endes gesehen werden. Bei der passiven Navigation hat sich insbesondere die elektromagnetische Navigation bewährt, bei der ein elektromagnetisches Feld extern um den Operationsbereich, beispielsweise durch einen Erzeuger eines elektromagnetischen Feldes in einem Kissen, auf dem der Patient liegt, erzeugt wird. Im chirurgischen Teil eingebaute spulenartige Sensoren ermöglichen die Ortung der Instrumente, woraufhin eine Darstellung in CT- oder MRT-Bildern erfolgen kann. Dieses Verfahren beinhaltet keine Strahlenbelastung und reduziert insgesamt so die Strahlenbelastung, auch durch einen reduzierten Röntgeneinsatz. Es wird weder die Bildqualität beeinträchtigt, noch können Sensoren, da es eben keine optischen Sensoren sind, verdeckt werden. Die Bewegungsfreiheit des Operateurs wird nicht eingeschränkt, wie es bei optischen Systemen der Fall ist. Die Arbeit des Operateurs wird wesentlich erleichtert.

Die US 2014/039258 A1, die als nächstliegender Stand der Technik angesehen wird, zeigt eine Endoskop-Einführform-Beobachtungssonde, umfassend: eine Spule mit einem ersten Isolationselement, das an einem vorderen Ende eines Kerns aus einem Magnetelement vorgesehen ist, und einem zweiten Isolationselement, das an einem hinteren Ende des Kerns vorgesehen ist; einen Spulenabschnitt, der durch Aufwickeln von Metallleitungen auf den Kern gebildet wird; mindestens zwei Anschlussabschnitte, mit denen die Metallleitungen elektrisch verbunden sind; und Signalleitungen mit Drähten, die elektrisch mit den Anschlussabschnitten verbunden sind und sich nach hinten erstrecken, während sie zwischen einer inneren Umfangsfläche eines Rohrs und einem äußeren Umfangsabschnitt des Spulenabschnitts angeordnet sind, wobei die Drähte an den beiden Anschlussabschnitten jeweils an den Oberflächenseiten davon gelötet sind. Die Oberflächenseiten sind in entgegengesetzten Richtungen von einander weg nach außen von jeder Magnetspuleneinheit gerichtet. Signalleitungen sind mit mehreren Magnetspuleneinheiten verbunden, ohne einen Außendurchmesser zu vergrößern. Eine praktikable sowohl elektrische als auch mechanische Verbindung zu einer Verarbeitungsansicht wird nicht offenbart. Auch die US5997473 A zeigt eine Sensoreinheit mit hintereinander angeordneten elektromagnetischen Sensoren zum Einsetzen in ein Endoskop, wobei auch hier eine fest vorgegebene Ausrichtung beider Sensoren nicht gegeben ist.

Der Erfindung liegt die Aufgabe zugrunde, unter Vermeidung der vorgenannten Nachteile eine praktische Vorrichtung zu schaffen, bei der insbesondere Ort und Orientierung eines Endkoskopes, insbesondere des distalen Endes präzise bestimmt werden kann.

Erfindungsgemäß wird die genannte Aufgabe mit einer Endoskop-Vorrichtung der eingangs genannten Art gelöst, welche die kennzeichnenden Merkmale des Anspruchs 1 aufweist.

Dadurch, dass die beiden Sensorspulen unter einem endlichen Winkel relativ zueinander in der Endoskop-Vorrichtung angeordnet sind, wird erreicht, dass aufgrund der unterschiedlichen Ausordnung die Orientierung der Endoskopvorrichtung im Magnetfeld des Erfassungssystems und damit auch im Raum präzise bestimmt werden kann.

Damit wird der Ort der einen Sensorspule, insbesondere der ersten Sensorspule in der Endoskop-Vorrichtung genau festgelegt und damit aufgrund der Bestimmung des Ortes der ersten Sensorspule durch das Erfassungssystem im Magnetfeld auch die exakte Bestimmung des genauen Ortes des distalen Endes der Sensor-Vorrichtung und damit des Arbeitsortes möglich, da der Abstand der (ersten) Sensorspule vom distalen Ende des Endoskopschaftes derart fest vorgegeben ist. Durch die feste Verbindung des die Sensorspule tragenden Stabs mit einem trennbaren Halter wird weiterhin erreicht, dass einerseits nach Positionierung der die die Sensorspule tragenden Stabs entfernt und dass das durch diesen zur Positionierung belegte Lumen für andere Einsatzzwecke freigegeben werden kann. Weiter ist so die durch den Stab mit den Sensorspulen gebildete Sensoreinrichtung vom eigentlichen Endoskop trennbar und kann anderweitig eingesetzt werden. Dies ermöglicht auch eine einfachere Sterilisation.

Weitere bevorzugte Ausgestaltungen der Erfindung sehen vor, dass die erste Sensorspule am distalen Ende des der Sensorspule tragenden Stabes angeordnet ist und/oder dass die zweite Spule in einem unter einem endlichen Winkel zur Mittelachse verlaufenden Ansatz des Einführkopfes mit ebenfalls einem endlichen Winkel zur Mittelachse angeordnet ist.

In bevorzugter Ausgestaltung ist dabei vorgesehen, dass die erste Sensorspule parallel zur Mittelachse ausgerichtet ist und die zweite Sensorspule unter einem endlichen Winkel zur Mittelachse ausgerichtet ist, wobei insbesondere die erste Sensorspule im Schaft angeordnet ist und die zweite Sensorspule in einem Ansatz des Einführkopfes angeordnet ist.

In Weiterbildung ist vorgesehen, dass der die beiden Sensorspulen tragende Stab sich durch ein Lumen der Vorrichtung erstreckt.

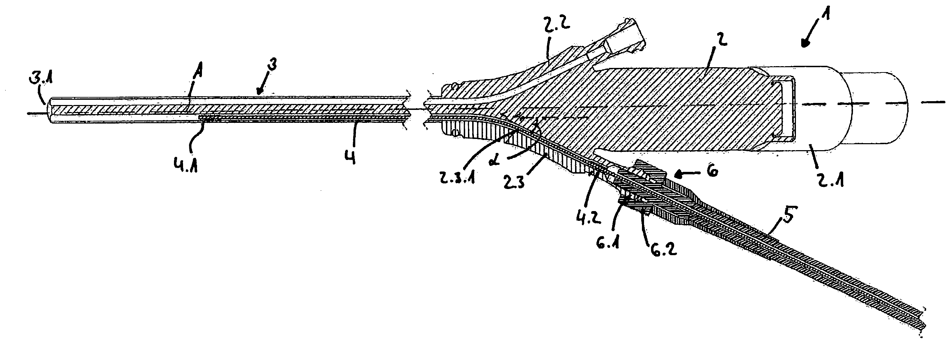

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnung im Einzelnen erläutert ist. dabei zeigt die einzige Figur
eine erfindungsgemäße Endoskop-Vorrichtung im Längsschnitt

Die erfindungsgemäße Endoskop-Vorrichtung 1 weist im dargestellten Ausführungsbeispiel einen proximalen Einführkopf 2 und einen sich von diesem distal erstreckenden Schaft 3 auf. Im dargestellten Ausführungsbeispiel weist der Einführkopf 2 drei Abzweigungen oder Ansätze 2.1 bis 2.3 auf, nämlich einen Ansatz 2.1 zur Einführung eines Lichtleiters vom proximalen Ende der Vorrichtung 1 bis zu deren distalen Ende sowie zwei Ansätze 2.2, 2.3 die ebenfalls jeweils ein Lumen oder einen Kanal aufweisen, der sich vom distalen Ende des gestreckten Schaftes 3 mit einer Mittelachse A bis zu einem Austritt des jeweiligen Adapters 2.2, 2.3.

Durch das Lumen 2.3.1 des Ansatzes 2.3 erstreckt sich ein Stab 4 der mit Abstand in seiner Längserstreckung zwei Spulen 4.1 und 4.2 trägt. Der Stab kann als massiver oder flexibler Stab oder aber als schraubenförmig gewendelter Stab ausgebildet sein. Von den Spulen 4.1, 4.2 erstreckt sich jeweils ein Anschlussdraht (nicht dargestellt) in proximaler Richtung bis zu einem jeweiligen proximalen Anschluss- oder Kontaktende des jeweiligen Drahtes, gegebenenfalls in der Ausbildung eines Steckers zum Anschluss der Drähte an eine Auswerteeinrichtung (nicht dargestellt).

Die Spule 4.1 findet sich am distalen Ende des Stabes 4 und damit innerhalb des parallel zu seiner Mittelachse A verlaufenden Schaftes 3 und ist daher ebenfalls parallel zur Mittelachse A oder achsparallel ausgerichtet. Die mit Abstand zur Spule 4.1 am Stab 4 angeordneten Spule 4.2 findet sich in dem unter einem endlichen Winkel zur Mittelachse A verlaufenden Ansatz 2.3 des Einführkopfes. In dem der Lumenabschnitt des Lumens 2.3.1 ebenfalls unter einem endlichen Winkel zur Mittelachse A verläuft, schließt die Ausrichtung oder Erstreckung der Spule 4.2 im Ansatz 2.3 ebenfalls einen endlichen Winkel zur Mittelachse A ein. Die beiden Spulen 4.1, 4.2 sind daher nicht parallel zueinander, sondern unter einem endlichen Winkel zueinander ausgerichtet.

Bei einem extern angelegten inhomogenen elektromagnetischen Feld, in dem sich die Spulen 4.1, 4.2 befinden, nehmen diese daher das Feld unterschiedlich wahr und senden an die Auswerteeinrichtung unterschiedliche Signale. Durch diese unterschiedliche Ausrichtung der Spulen 4.1, 4.2 kann daher die Orientierung der Endoskop-Vorrichtung im elektromagnetischen Feld und damit im Raum exakt bestimmt werden.

Der Stab 4 ist in einem Halter 5 axial fest angeordnet. Der Halter 5 und der Ansatz 2.3 sind miteinander durch einen Luer-Adapter 6 verbindbar, wobei jedes der Teile einen jeweils zueinander korrespondierenden Teil des 6.1, 6.2 des Luer-Adapters 6 trägt, so dass der Ansatz 2.3 und der Halter 5 durch den Luer-Adapter 6 in an sich bekannter Weise bajonettartig fest miteinander verbunden werden können. Damit ist der Ort des Halters 5 relativ zum Ansatz 2.3 und auch zum Kopf 2 sowie zum Schaft 3 der Endoskop-Vorrichtung 1 im befestigten Zustand definiert. Da der Schaft 4, wie gesagt, axial fest im Halter 5 angeordnet ist, ist damit auch die Längsposition der Spulen 4.1, 4.2 und insbesondere der distalen Spule 4.1 im Schaft 3 und damit der Endoskop-Vorrichtung 1 festgelegt und damit auch der Abstand insbesondere der axiale Abstand der Spule 4.1 zum distalen Ende 3.1 des Schaftes 3. Damit kann durch das Sensorsignal der Spule 4.1 im angelegten elektromagnetischen Feld auch deren Ort und aufgrund des festen Abstandes zum distalen Ende 3.1 des Schaftes 3 der Ort des distalen Endes 3.1 des Schaftes 3 im elektromagnetischen Feld und damit im Raum bestimmt werden.

Damit erkennt ein Operateur aufgrund der Auswertung der Sensorsignale und einer Bildanzeige auf einem Bildschirm der Auswerteeinrichtung genau die Position des distalen Endes 3.1 des Schaftes 3 und damit auch der Endoskop-Vorrichtung 1 und weiß damit wo er exakt mit seinen Instrumenten, die er gegebenenfalls durch anderen Lumen der Endoskop-Vorrichtung ein und durch diese hindurchführt, arbeitet.

## Patentansprüche

1. Endoskop-Vorrichtung (1) mit einem proximalen Einführkopf (2) und mit einem sich von diesem distal erstreckendem Schaft (3) mit einer Mittelachse (A), mit mindestens einem sich durch die Vorrichtung (1) erstreckenden länglichen Lumen (2.3.1), mit einem Sensorstab (4) mit mindestens zwei in Längsrichtung mit endlichem Abstand zu einander angeordneten Sensorspulen (4.1,4.2), die relativ zueinander unter einem endlichen Winkel (L) ausgerichtet sind, **dadurch gekennzeichnet, dass** der Stab (4) axial fest mit einem auf den Ansatz (2.3) des Einführkopfes (2) aufsetzbaren Halter (5) verbunden ist und dass der Ansatz (2.3) des Einführkopfes (2) und der Halter (5) einander zugewandte korrespondierende Luer-Adapter-Teile (6.1, 6.2) mit Luer-Adapter (6) aufweisen und Ansatz (2.3) und Halter (5) durch den Luer-Adapter (6) fest miteinander verbindbar sind.

2. Vorrichtung nach Anspruch 1, **dadurch gekennzeichnet, dass** die erste Sensorspule (4.1) parallel zur Mittelachse (A) ausgerichtet ist und die zweite Sensorspule unter einem endlichen Winkel (L) zur Mittelachse ausgerichtet ist.

3. Vorrichtung nach Anspruch 1 oder 2, **dadurch gekennzeichnet, dass** die erste Sensorspule (4.1) im Schaft (3) angeordnet ist.

4. Vorrichtung nach einem der vorangehenden Ansprüche, **dadurch gekennzeichnet, dass** die zweite Sensorspule (4.2) in einem Ansatz (2.3) des Einführkopfes (2) angeordnet ist.

5. Vorrichtung nach einem der vorangehenden Ansprüche, **dadurch gekennzeichnet, dass** der die beiden Sensorspulen (4.1, 4.2) tragende Stab (4) sich durch ein Lumen (2.3.1) der Vorrichtung (1) erstreckt.

6. Vorrichtung nach einem der vorangehenden Ansprüche, **dadurch gekennzeichnet, dass** von jeder Sensorspule (4.1, 4.2) aus sich ein Anschlussdraht durch den Stab (4) bis zu dessen proximalen Ende zum Anschluss an eine Auswerteeinrichtung erstreckt.

7. Vorrichtung nach einem der vorangehenden Ansprüche, **dadurch gekennzeichnet, dass** die erste Sensorspule (4.1) am distalen Ende des die Sensorspule (4.1) tragenden Stabes (4) angeordnet ist.

8. Vorrichtung nach einem der vorangehenden Ansprüche, **dadurch gekennzeichnet, dass** die zweite Spule (4.2) in einem unter einem endlichen Winkel zur Mittelachse (A) verlaufenden Ansatz (2.3) des Einführkopfes (5.1) mit ebenfalls einem endlichen Winkel zur Mittelachse (A) angeordnet ist.

## Claims

1. Endoscope device (1) having a proximal insertion head (2) and having a shaft (3) extending distally therefrom having a centre axis (A), having at least one oblong lumen (2.3.1) extending through the device (1), with a sensor rod (4) having at least two sensor coils (4.1, 4.2) arranged at a finite spacing in relation to one another in the longitudinal direction, which are oriented in relation to one another at a finite angle (L) **characterized in that** the rod (4) is connected in an axially fixed manner to a holder (5) attachable to the attachment (2.3) of the insertion head (2) and that the attachment (2.3) of the insertion head (2) and the holder (5) comprise corresponding Luer adapter parts (6.1, 6.2) facing toward one another having Luer adapter (6), and attachment (2.3) and holder (5) are fixedly connectable to one another by the Luer adapter (6) .

2. Device according to Claim 1, **characterized in that** the first sensor coil (4.1) is oriented in parallel to the centre axis (A) and the second sensor coil is oriented at a finite angle (L) in relation to the centre axis.

3. Device according to Claim 1 or 2, **characterized in that** the first sensor coil (4.1) is arranged in the shaft (3) .

4. Device according to one of the preceeding claims, **characterized in that** the second sensor coil (4.2) is arranged in an attachment (2.3) of the insertion head (2) .

5. Device according to one of the preceeding claims, **characterized in that** the rod (4) bearing the two sensor coils (4.1, 4.2) extends through a lumen (2.3.1) of the device (1).

6. Device according to one of the preceeding claims, **characterized in that** a connecting wire extends from each sensor coil (4.1, 4.2) through the rod (4) up to its proximal end for connection to an analysis unit.

7. Device according to one of the preceeding claims, **characterized in that** the first sensor coil (4.1) is arranged at the distal end of the rod (4) which bears the sensor coil (4.1).

8. Device according to one of the preceeding claims, **characterized in that** the second coil (4.2) is arranged at a finite angle to the centre axis (A) in an attachment (2.3) oft he insertion head (5.1) which extends at a finite angle to the central axis (A).

## Revendications

1. Dispositif endoscopique (1) équipé d'une tête d'introduction proximale (2) et d'une tige (3) s'étendant dans le plan distal'depuis ladite tête avec un axe central (A), avec au moins une lumière (2.3.1) oblongue s'étendant à travers le dispositif (1), avec une barre de détection (4) avec au moins deux bobines de détection (4.1, 4.2) disposées à une certaine distance finie l'une de l'autre dans la direction longitudinale et orientées l'une par rapport à l'autre selon un angle fini (L), **caractérisé en ce que** la barre (4) est reliée fixement dans le plan axial à un support (5) pouvant être placé sur l'appendice (2.3) de la tête d'introduction (2) et que l'appendice (2.3) de la tête d'introduction (2) et le support (5) comportent des parties d'adaptateur Luer (6.1, 6.2) correspondantes orientées l'une vers l'autre, avec adaptateur Luer (6), et que l'appendice (2.3) et le support (5) peuvent être reliés fixement entre eux par l'adaptateur Luer (6).

2. Dispositif selon la revendication 1, **caractérisé en ce que** la première bobine de détection (4.1) est orientée parallèlement à l'axe central (A) et que la deuxième bobine de détection est orientée selon un angle fini (L) par rapport à l'axe central.

3. Dispositif selon la revendication 1 ou 2, **caractérisé en ce que** la première bobine de détection (4.1) est disposée dans la tige (3).

4. Dispositif selon l'une quelconque des revendications précédentes, **caractérisé en ce que** la deuxième bobine de détection (4.2) est disposée dans un appendice (2.3) de la tête d'introduction (2).

5. Dispositif selon l'une quelconque des revendications précédentes, **caractérisé en ce que** la barre (4) supportant les deux bobines de détection (4.1, 4.2) s'étend à travers une lumière (2.3.1) du dispositif (1).

6. Dispositif selon l'une quelconque des revendications précédentes, **caractérisé en ce que** depuis chaque bobine de détection (4.1, 4.2), un fil de raccordement s'étend à travers la barre (4) jusqu'à son extrémité proximale pour le raccordement à un dispositif d'analyse.

7. Dispositif selon l'une quelconque des revendications précédentes, **caractérisé en ce que** la première bobine de détection (4.1) est disposée au niveau de l'extrémité distale de la barre (4) supportant la bobine de détection (4.1).

8. Dispositif selon l'une quelconque des revendications précédentes, **caractérisé en ce que** la deuxième bobine (4.2) est disposée dans un appendice (2.3), s'étendant selon un angle fini par rapport à l'axe central (A), de la tête d'introduction (5.1) avec également un angle fini par rapport à l'axe central (A).
